# EUROPEAN PATENT APPLICATION

(11) **EP 3 832 656 A1**
(43) Date of publication of application: **09.06.2021**
(21) Application number: 18927301.4
(22) Date of filing: 27.07.2018
(51) Int. Cl.: G16B 15/00

(54) **COLLECTIVE COORDINATE DETERMINATION METHOD, DETERMINATION DEVICE, AND PROGRAM**

(71) Applicant: FUJITSU LIMITED, Kanagawa 211-8588 (JP)
(72) Inventor: SATO, Hiroyuki, Kawasaki-shi, Kanagawa 211-8588 (JP)
(74) Representative: Haseltine Lake Kempner LLP
(86) International application number: PCT/JP2018/028317
(87) International publication number: WO 2020/021719

(57) **Abstract**

A method of determining a collective coordinate by using a computer includes
a process of obtaining a binding site of a binding calculation object molecule in a target molecule,
a process of obtaining a normal direction (N1),
a process of obtaining an oriented binding calculation object molecule,
a process of selecting an atom (A1) having a minor fluctuation when the binding site is obtained,
a process of selecting an atom (A2) having a minor fluctuation when the binding site is obtained,
a process of obtaining a center (A3) of the ring structure,
a process of selecting an atom (A4) that does not overlap with the center (A3), and
a process of determining a dihedral angle formed by a plane formed by the atom (A1), the center (A3), and the atom (A4) and a plane formed by the atom (A1), the atom (A2), and the center (A3) as a collective coordinate.

## Description

### TECHNICAL FIELD

This case relates to a method of determining a collective coordinate, a device of determining a collective coordinate, and a program of determining a collective coordinate.

### BACKGROUND ART

In a case where a certain protein has a functional site that adversely affects the body, it is necessary to design a ligand that stably binds to the functional site of the protein in drug discovery targeting the protein. By stably binding the ligand, the functional site of the protein is blocked, and consequently, this causes suppression of the adverse effect on the body.

A technology is reported that predicts binding free energy by information technology (IT) when binding stability between the protein and the ligand is determined with the same accuracy as an experiment (for example, refer to Patent Document 1). However, three-dimensional information of a complex obtained by binding a ligand in an appropriate pose is needed for the present technology to be effective. In order to efficiently search for the appropriate binding pose (stable binding structure) of the ligand by metadynamics or the like, a collective coordinate (synonymous with collective variable (CV)) capable of setting an appropriate search space is needed. However, information obtained by the experiments such as a three-dimensional crystal structure of a binding site, or one binding pose of the ligand in the site, is needed for setting of the CV.

### CITATION LIST

### PATENT DOCUMENT

Patent Document 1: Japanese Laid-open Patent Publication No. 2006-209764

### SUMMARY OF INVENTION

### TECHNICAL PROBLEM

A problem of this case is to solve the traditional problems and to achieve the following object. In other words, an object of this case is to provide a method of determining a collective coordinate, a collective coordinate determination device, and a program of determining a collective coordinate that can obtain a collective coordinate capable of setting a search space of a stable binding structure when a target molecule is bound to a binding calculation object molecule without using experimental information.

### SOLUTION TO PROBLEM

Means for solving the problems is as follows. In other words,
the method of determining a collective coordinate according to the disclosure is a method of determining a collective coordinate, by using a computer, that determines a collective coordinate capable of setting a search space of a stable binding structure when a target molecule is bound to a binding calculation object molecule, including:
a process of obtaining a binding site of the binding calculation object molecule in the target molecule by molecular dynamics by using a fragment, including a ring structure, that is a part or all of the binding calculation object molecule;
a process of obtaining a normal direction (N1) with respect to a plane obtained from the ring structure of the fragment at coordinates of the fragment where the fragment frequently appears in the binding site when the binding site is obtained by the molecular dynamics;
a process of obtaining the binding calculation object molecule that is in the binding site and does not overlap with the target molecule, the binding calculation object molecule being oriented so that a normal direction (N2) with respect to a plane obtained from the ring structure of the fragment of the binding calculation object molecule is overlapped with the normal direction (N1);
a process of selecting an atom (A1) of the target molecule in a columnar region obtained by moving the ring structure of the obtained oriented binding calculation object molecule to the normal direction (N2), the atom (A1) having a minor fluctuation when the binding site is obtained by the molecular dynamics;
a process of selecting an atom (A2) of the target molecule existing on a surface including a plane obtained from the ring structure of the obtained oriented binding calculation object molecule, the atom (A2) having a minor fluctuation when the binding site is obtained by the molecular dynamics;
a process of obtaining a center (A3) of the ring structure of the obtained oriented binding calculation object molecule;
a process of selecting an atom (A4) that does not overlap with the center (A3) from among atoms included in the fragment of the obtained oriented binding calculation object molecule; and
a process of determining a dihedral angle formed by a plane formed by the atom (A1), the center (A3), and the atom (A4) and a plane formed by the atom (A1), the atom (A2), and the center (A3) as a collective coordinate.

A program according to the disclosure is a program for causing a computer to determine a collective coordinate capable of setting a search space of a stable binding structure when a target molecule is bound to a binding calculation object molecule, the program causing a computer to execute:
a process of obtaining a binding site of the binding calculation object molecule in the target molecule by molecular dynamics by using a fragment, including a ring structure, that is a part or all of the binding calculation object molecule;
a process of obtaining a normal direction (N1) with respect to a plane obtained from the ring structure of the fragment at coordinates of the fragment where the fragment frequently appears in the binding site when the binding site is obtained by the molecular dynamics;
a process of obtaining a binding calculation object molecule that is in the binding site and does not overlap with the target molecule, the binding calculation object molecule being oriented so that a normal direction (N2) with respect to a plane obtained from the ring structure of the fragment of the binding calculation object molecule is overlapped with the normal direction (N1);
a process of selecting an atom (A1) of the target molecule in a columnar region obtained by moving the ring structure of the obtained oriented binding calculation object molecule to the normal direction (N2), the atom (A1) having a minor fluctuation when the binding site is obtained by the molecular dynamics;
a process of selecting an atom (A2) of the target molecule existing on a surface including a plane obtained from the ring structure of the obtained oriented binding calculation object molecule, the atom (A2) having a minor fluctuation when the binding site is obtained by the molecular dynamics;
a process of obtaining a center (A3) of the ring structure of the obtained oriented binding calculation object molecule;
a process of selecting an atom (A4) that does not overlap with the center (A3) from among atoms included in the fragment of the obtained oriented binding calculation object molecule; and
a process of determining a dihedral angle formed by a plane formed by the atom (A1), the center (A3), and the atom (A4) and a plane formed by the atom (A1), the atom (A2), and the center (A3) as a collective coordinate.

A collective coordinate determination device according to the disclosure is a collective coordinate determination device that determines a collective coordinate capable of setting a search space of a stable binding structure when a target molecule is bound to a binding calculation object molecule, including:
a binding site search unit that obtains a binding site of the binding calculation object molecule in the target molecule by molecular dynamics by using a fragment, including a ring structure, that is a part or all of the binding calculation object molecule;
a normal direction (N1) search unit that obtains a normal direction (N1) with respect to a plane obtained from the ring structure of the fragment at coordinates of the fragment where the fragment frequently appears in the binding site when the binding site is obtained by the molecular dynamics;
a binding calculation object molecule search unit that obtains the binding calculation object molecule that is in the binding site and does not overlap with the target molecule, the binding calculation object molecule being oriented so that a normal direction (N2) with respect to a plane obtained from the ring structure of the fragment of the binding calculation object molecule is overlapped with the normal direction (N1);
an atom (A1) selection unit that selects an atom (A1) of the target molecule in a columnar region obtained by moving the ring structure of the obtained oriented binding calculation object molecule to the normal direction (N2), the atom (A1) having a minor fluctuation when the binding site is obtained by the molecular dynamics;
an atom (A2) selection unit that selects an atom (A2) of the target molecule existing on a surface including a plane obtained from the ring structure of the obtained oriented binding calculation object molecule, the atom (A2) having a minor fluctuation when the binding site is obtained by the molecular dynamics;
a center (A3) search unit that obtains a center (A3) of the ring structure of the obtained oriented binding calculation object molecule;
an atom (A4) selection unit that selects an atom (A4) that does not overlap with the center (A3) from among atoms included in the fragment of the obtained oriented binding calculation object molecule; and
a collective coordinate determination unit that determines a dihedral angle formed by a plane formed by the atom (A1), the center (A3), and the atom (A4) and a plane formed by the atom (A1), the atom (A2), and the center (A3) as a collective coordinate.

### ADVANTAGEOUS EFFECTS OF INVENTION

According to a method of determining a collective coordinate according to the disclosure, it is possible to solve the conventional problems and achieve the object, and it is possible to obtain a collective coordinate capable of setting a search space of a stable binding structure when a target molecule is bound to a binding calculation object molecule without using experimental information.

According to a program according to the disclosure, it is possible to solve the conventional problems and achieve the object, and it is possible to obtain a collective coordinate capable of setting a search space of a stable binding structure when a target molecule is bound to a binding calculation object molecule without using experimental information.

According to a collective coordinate determination device according to the disclosure, it is possible to solve the conventional problems and achieve the object, and it is possible to obtain a collective coordinate capable of setting a search space of a stable binding structure when a target molecule is bound to a binding calculation object molecule without using experimental information.

### BRIEF DESCRIPTION OF DRAWINGS

FIG. 1 is a flowchart of an example of a method of determining a collective coordinate according to the disclosure.
FIG. 2A is a conceptual diagram for explaining an example of the method of determining the collective coordinate according to the disclosure (No. 1).
FIG. 2B is a conceptual diagram for explaining an example of the method of determining the collective coordinate according to the disclosure (No. 2).
FIG. 2C is a conceptual diagram for explaining an example of the method of determining the collective coordinate according to the disclosure (No. 3).
FIG. 2D is a conceptual diagram for explaining an example of the method of determining the collective coordinate according to the disclosure (No. 4).
FIG. 2E is a conceptual diagram for explaining an example of the method of determining the collective coordinate according to the disclosure (No. 5).
FIG. 2F is a conceptual diagram for explaining an example of the method of determining the collective coordinate according to the disclosure (No. 6).
FIG. 2G is a conceptual diagram for explaining an example of the method of determining the collective coordinate according to the disclosure (No. 7).
FIG. 2H is a conceptual diagram for explaining an example of the method of determining the collective coordinate according to the disclosure (No. 8).
FIG. 2I is a conceptual diagram for explaining an example of the method of determining the collective coordinate according to the disclosure (No. 9).
FIG. 2J is a conceptual diagram for explaining an example of the method of determining the collective coordinate according to the disclosure (No. 10).
FIG. 2K is a conceptual diagram for explaining an example of the method of determining the collective coordinate according to the disclosure (No. 11).
FIG. 3 is an exemplary configuration of a collective coordinate determination device according to the disclosure.
FIG. 4 is another exemplary configuration of the collective coordinate determination device according to the disclosure.
FIG. 5 is still another exemplary configuration of the collective coordinate determination device according to the disclosure.
FIG. 6 is a diagram illustrating a result of a first embodiment.

### DESCRIPTION OF EMBODIMENTS

### (Method of Determining Collective Coordinate)

A method of determining a collective coordinate according to the disclosure is performed by using a computer.

The method of determining the collective coordinate determines a collective coordinate (synonymous with collective variables (CV)) capable of setting a search space of a stable binding structure when a binding calculation object molecule is bound to a target molecule.

The method of determining the collective coordinate includes at least a process of obtaining a binding site, a process of obtaining a normal direction (N1), a process of obtaining an oriented binding calculation object molecule, a process of selecting an atom (A1), a process of selecting an atom (A2), a process of obtaining a center (A3), a process of selecting an atom (A4), and a process of determining a dihedral angle as a collective coordinate, and further includes other processes as needed.

### <Process of Obtaining Binding Site>

In the process of obtaining the binding site, the binding site of the binding calculation object molecule in the target molecule is obtained by molecular dynamics by using a fragment which is a part or all of the binding calculation object molecule and includes a ring structure.

The target molecule is not particularly limited and can be appropriately selected according to a purpose. Examples of the target molecule include a protein, a ribonucleic acid (RNA), a deoxyribonucleic acid (DNA), and the like.

The binding calculation object molecule means a drug candidate molecule or a fragment molecule when the drug candidate molecule is designed.

The fragment molecule is used, for example, for fragment based drug design (FBDD).

The binding calculation object molecule used for the technology according to the disclosure has a ring structure.

The ring structure is not particularly limited and can be appropriately selected according to a purpose. For example, the ring structure may be an aromatic ring or may be a non-aromatic ring. Furthermore, the ring structure may be a homocyclic ring or a heterocyclic ring. Furthermore, the ring structure may be monocyclic or polycyclic.

In a case where a structure of the binding calculation object molecule is large, for example, a fragment including a ring structure is selected from a part of the binding calculation object molecule.

In a case where the structure of the binding calculation object molecule is small, the binding calculation object molecule itself may be used as a fragment.

The fragment used for the method of determining the collective coordinate may be one type or a plurality of types of fragment as long as a fragment includes the ring structure and is selected from the binding calculation object molecule.

In a case where there is the plurality of ring structures in the binding calculation object molecule, the ring structure in the fragment is preferably a ring structure at an end of the binding calculation object molecule. This is because the ring structure at the end of the binding calculation object molecule among the ring structures in the binding calculation object molecule is more likely to enter the binding site.

For example, in a case where three ring structures are included in the binding calculation object molecule and the three ring structures are aligned in a line, it is preferable to include one of the ring structures at both ends in the fragment.

A program used for the molecular dynamics is not particularly limited and can be appropriately selected according to a purpose. For example, the program includes Groningen Machine for Chemical Simulations (gromacs), Assisted Model Building with Energy Refinement (amber), charmm, tinker, lammps, or the like.

The method of obtaining the binding site of the binding calculation object molecule in the target molecule by the molecular dynamics is not particularly limited and can be appropriately selected according to a purpose. For example, the method includes a method of performing the molecular dynamics on the target molecule in a fragment solution including the plurality of fragments and assuming a region of the target molecule where the fragments exist at a relatively high concentration during the molecular dynamics as a binding site, and the like.

The concentration of the fragment in the fragment solution is not particularly limited and can be appropriately selected according to a purpose as long as the concentration allows the molecular dynamics to proceed smoothly. For example, the concentration is 1 M (1 mol/L) or the like.

### <Process of Obtaining Normal Direction (N1)>

In the process of obtaining the normal direction (N1), a normal direction (N1) with respect to a plane obtained from the ring structure of the fragment existing at coordinates where the fragment frequently appears in the binding site when the binding site is obtained by the molecular dynamics is obtained.

In the process of obtaining the normal direction (N1), for example, first, the coordinates of the fragment where the fragment frequently appears in the binding site when the binding site is obtained by the molecular dynamics are obtained. Subsequently, the normal direction (N1) with respect to the plane obtained from the ring structure of the fragment at the coordinates is obtained.

The coordinates are, for example, coordinates where a number of fragments exist in the binding site when a plurality of snapshots obtained by the molecular dynamics is superimposed. Here, the coordinates where a number of fragments exist do not mean that a number of fragments take exactly the same coordinates, and the coordinates of a number of fragments may be different from each other within a predetermined allowable range. In other words, the coordinates may be obtained by clustering the coordinates that can be assumed to be the same within the predetermined allowable range. The predetermined allowable range is not particularly limited and can be appropriately selected according to a purpose.

In a case where the ring structure has a planar structure, the plane is a plane including the planar structure.

In a case where the ring structure does not have a planar structure, the plane is, for example, a plane having the smallest total distance from each heavy atom included in the ring structure.

Here, the heavy atom is an atom other than a hydrogen atom.

### <Process of Obtaining Oriented Binding Calculation Object Molecule>

In the process of obtaining the oriented binding calculation object molecule, the binding calculation object molecule which is in the binding site and does not overlap with the target molecule, the binding calculation object molecule being oriented so that a normal direction (N2) with respect to the plane obtained from the ring structure of the fragment of the binding calculation object molecule is overlapped with the normal direction (N1), is obtained.

Here, "the normal direction (N2) is overlapped with the normal direction (N1)" does not mean only that the normal directions completely coincide with each other and includes that the normal directions substantially coincide with each other. In other words, the normal direction (N2) and the normal direction (N1) may deviate from perfect coincidence within a predetermined allowable range. The predetermined allowable range is not particularly limited and can be appropriately selected according to a purpose.

Note that, in a case where the fragment is the entire binding calculation object molecule, the fragment coincides with the binding calculation object molecule. Therefore, the process of obtaining the oriented binding calculation object molecule has been already completed by obtaining the coordinates where the fragment frequently appears in the process of obtaining the normal direction (N1) .

The target molecule does not overlap with the binding calculation object molecule means that, in a coordinate space, the target molecule in the coordinate space does not overlap with the binding calculation object molecule in the coordinate space.

The target molecule in the coordinate space is created from coordinates of each atom of the target molecule.

The binding calculation object molecule in the coordinate space is created from coordinates of each atom of the binding calculation object molecule.

The process of obtaining the oriented binding calculation object molecule is performed, for example, as follows.

The binding calculation object molecule is arranged so that the coordinates of the fragment where the fragment frequently appears in the process of obtaining the normal direction (N1) coincide with coordinates of the fragment. With this arrangement, the normal direction (N2) and the normal direction (N1) overlap with each other. Next, the binding calculation object molecule is rotated around the normal direction (N2) as a rotation axis, and coordinates where the binding calculation object molecule does not overlap with the target molecule are obtained. With this operation, by rotating the binding calculation object molecule in a state where the normal direction (N2) is overlapped with the normal direction (N1), it is possible to obtain coordinates where the binding calculation object molecule does not overlap with the target molecule.

The coincidence does not mean only complete coincidence and includes substantial coincidence. In other words, the binding calculation object molecule may be arranged so that the coordinates of the fragment coincide with the coordinates of the fragment where the fragment frequently appears within a predetermined allowable range. The predetermined allowable range is not particularly limited and can be appropriately selected according to a purpose. For example, a predetermined range can be determined on the basis of coordinates, angles, or the like.

### <Process of Selecting Atom (A1)>

In the process of selecting the atom (A1), an atom (A1) of the target molecule in a columnar region obtained by moving the ring structure of the obtained oriented binding calculation object molecule to the normal direction (N2), the atom (A1) having a minor fluctuation when the binding site is obtained by the molecular dynamics, is selected.

The atom (A1) is an atom of the target molecule in the columnar region obtained by moving the ring structure of the obtained oriented binding calculation object molecule to the normal direction (N2).

Furthermore, the atom (A1) is also an atom having a minor fluctuation when the binding site is obtained by the molecular dynamics.

The fluctuation of the atom having the minor fluctuation when the atom (A1) is selected is not particularly limited and can be appropriately selected according to a purpose. For example, the fluctuation includes a fluctuation minor than vibration caused by heat or the like. Furthermore, for example, the fluctuation includes a fluctuation equal to or less than two Å or the like. The fluctuation minor than the vibration caused by heat and the fluctuation equal to or less than two Å are usually minor than the fluctuations of many other atoms of the target molecule.

The vibration caused by heat is a vibration caused by heat at 25°C.

The fluctuation can be obtained by using, for example, Root Mean Square Deviation.

### <Process of Selecting Atom (A2)>

In the process of selecting the atom (A2), an atom (A2) of the target molecule existing on a surface including the plane obtained from the ring structure of the obtained oriented binding calculation object molecule, the atom (A2) having a minor fluctuation when the binding site is obtained by the molecular dynamics, is selected.

The atom (A2) is an atom of the target molecule existing on the surface including the plane obtained from the ring structure of the obtained oriented binding calculation object molecule.

Furthermore, the atom (A2) is also an atom having a minor fluctuation when the binding site is obtained by the molecular dynamics.

The fluctuation of the atom having the minor fluctuation when the atom (A2) is selected is not particularly limited and can be appropriately selected according to a purpose. For example, the fluctuation includes a fluctuation minor than vibration caused by heat or the like. Furthermore, for example, the fluctuation includes a fluctuation equal to or less than two Å or the like. The fluctuation minor than the vibration caused by heat and the fluctuation equal to or less than two Å are usually minor than the fluctuations of many other atoms of the target molecule.

The vibration caused by heat is a vibration caused by heat at 25°C.

The fluctuation can be obtained by using, for example, Root Mean Square Deviation.

### <Process of Obtaining Center (A3)>

In the process of obtaining the center (A3), a center (A3) of the ring structure of the obtained oriented binding calculation object molecule is obtained.

A method of obtaining the center (A3) is not particularly limited and can be appropriately selected according to a purpose. For example, the center (A3) may be obtained as a barycenter of coordinates of each heavy atom included in the ring structure or may be obtained as an arithmetic mean value of the coordinates of each heavy atom included in the ring structure.

The center (A3) may be on a plane obtained from the ring structure or may not be on the plane.

It is preferable that the center (A3) be in a surface obtained by projecting the ring structure on the plane obtained from the ring structure.

The center (A3) may be, for example, the center in the surface obtained by projecting the ring structure on the plane obtained from the ring structure.

### <Process of Selecting Atom (A4)>

In the process of selecting the atom (A4), an atom (A4) that does not overlap with the center (A3) is selected from among the atoms included in the fragment of the obtained oriented binding calculation object molecule.

The atom (A4) is an atom selected from among the atoms included in the fragment of the obtained oriented binding calculation object molecule.

The coordinates of the atom (A4) do not overlap with the coordinates of the center (A3).

### <Process of Determining Dihedral Angle as Collective Coordinate>

In the process of obtaining the dihedral angle as a collective coordinate, a dihedral angle formed by a plane formed by the atom (A1), the center (A3), and the atom (A4) and a plane formed by the atom (A1), the atom (A2), and the center (A3) is determined as a collective coordinate.

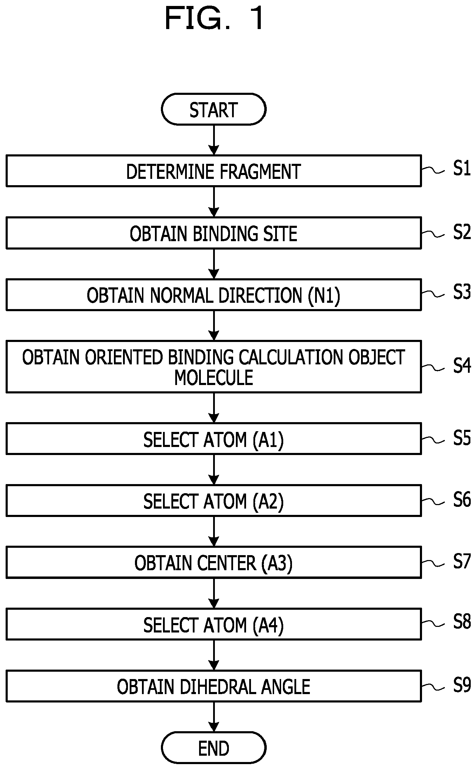

Here, an example of the technology according to the disclosure will be described with reference to the conceptual diagram and the flowchart illustrated in FIG. 1.

FIG. 1 is a flowchart of an example of a method of determining the collective coordinate according to the disclosure.

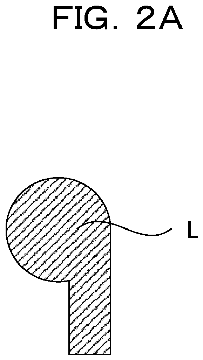

FIGs. 2A to 2K are conceptual diagrams for explaining an example of the method of determining the collective coordinate according to the disclosure.

### <<Step S1>>

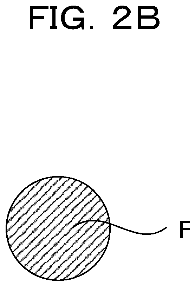

First, in step S1, a fragment used for the method of determining the collective coordinate is determined from a binding calculation object molecule. For example, a fragment F including a ring structure (FIG. 2B) that is a part of a binding calculation object molecule L illustrated in FIG. 2A is determined.

### <<Step S2>>

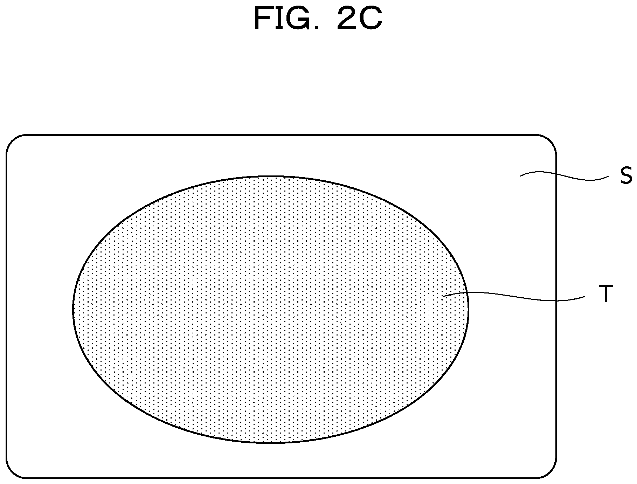

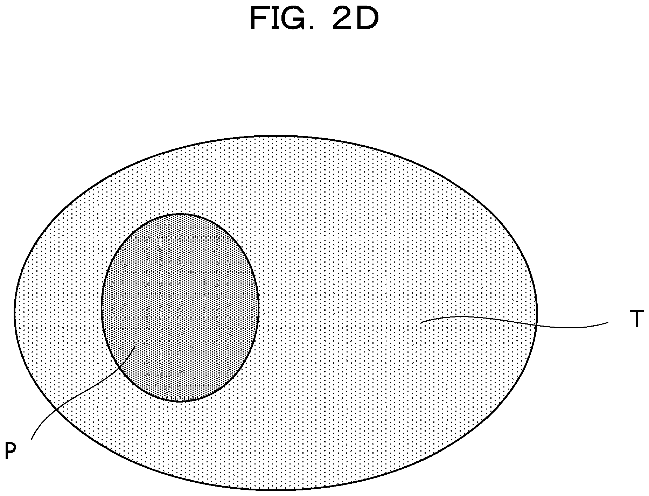

Next, in step S2, a binding site of the binding calculation object molecule L in a target molecule T is obtained by molecular dynamics (MD) by using the fragment F. For example, molecular dynamics is performed on the target molecule T in a fragment solution S including the plurality of fragments F, and a region of the target molecule T where the fragments F exist at a relatively high concentration during the molecular dynamics is assumed as a binding site P (FIGs. 2C and 2D).

### <<Step S3>>

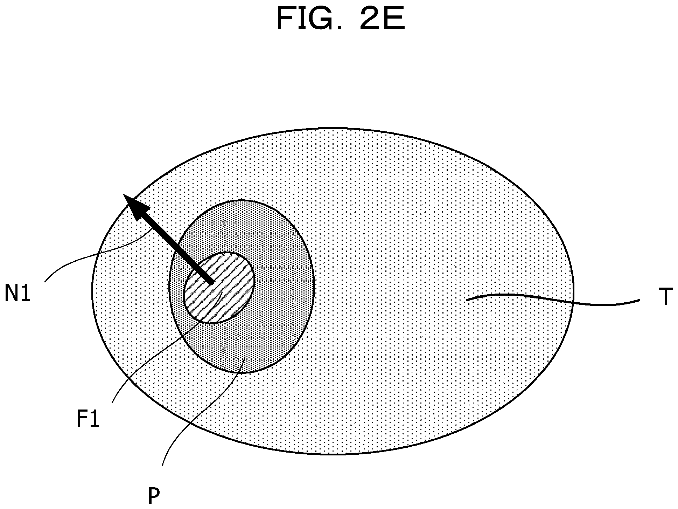

Next, in step S3, a normal direction (N1) with respect to a plane obtained from the ring structure of the fragment F1 at the coordinates of the fragment F where the fragment F frequently appears in the binding site P when the binding site is obtained by the molecular dynamics is obtained. For example, first, the coordinates of the fragment where the fragment F frequently appears in the binding site P when the binding site P is obtained by the molecular dynamics are obtained. Subsequently, the normal direction (N1) with respect to the plane obtained from the ring structure of the fragment F1 at the coordinates is obtained (FIG. 2E).

### <<Step S4>>

Next, in step S4, a binding calculation object molecule L1 which is in the binding site P and does not overlap with the target molecule T, the binding calculation object molecule L1 being oriented so that a normal direction (N2) with respect to the plane obtained from the ring structure of the fragment of the binding calculation object molecule L1 is overlapped with the normal direction (N1), is obtained.

More specifically, step S4 is performed, for example, as follows.

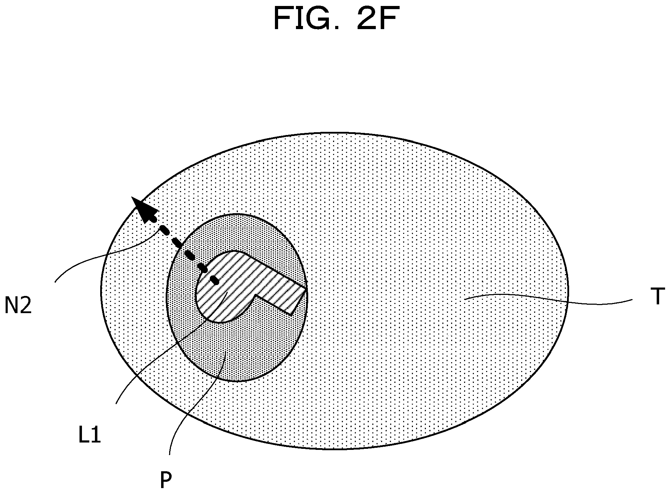

The binding calculation object molecule is arranged so that the coordinates of the fragment where the fragment frequently appears in the process of obtaining the normal direction (N1) coincide with coordinates of the fragment. With this arrangement, the normal direction (N2) and the normal direction (N1) overlap with each other. Next, the binding calculation object molecule L is rotated around the normal direction (N2) as a rotation axis, and coordinates where the binding calculation object molecule does not overlap with the target molecule are obtained. With this operation, by rotating the binding calculation object molecule in a state where the normal direction (N2) is overlapped with the normal direction (N1), it is possible to obtain coordinates where the binding calculation object molecule does not overlap with the target molecule. As a result, the oriented binding calculation object molecule L1 is obtained (FIG. 2F).

### <<Step S5>>

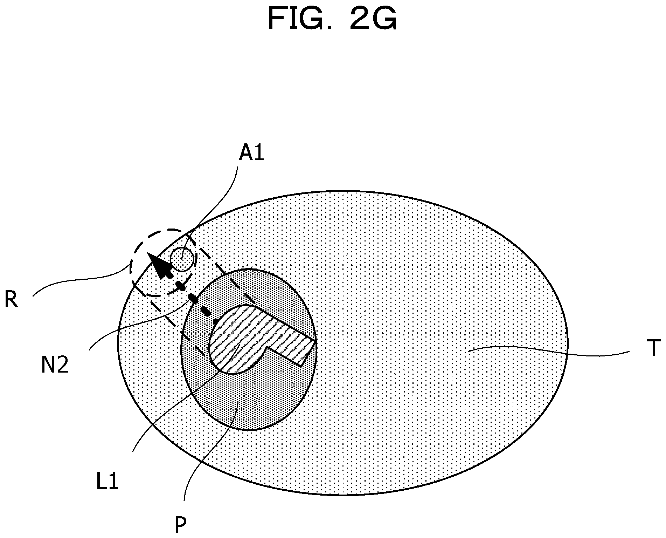

Next, in step S5, an atom (A1) of the target molecule in a columnar region R obtained by moving the ring structure of the obtained oriented binding calculation object molecule to the normal direction (N2), the atom (A1) having a minor fluctuation when the binding site is obtained by the molecular dynamics, is selected (FIG. 2G).

### <<Step S6>>

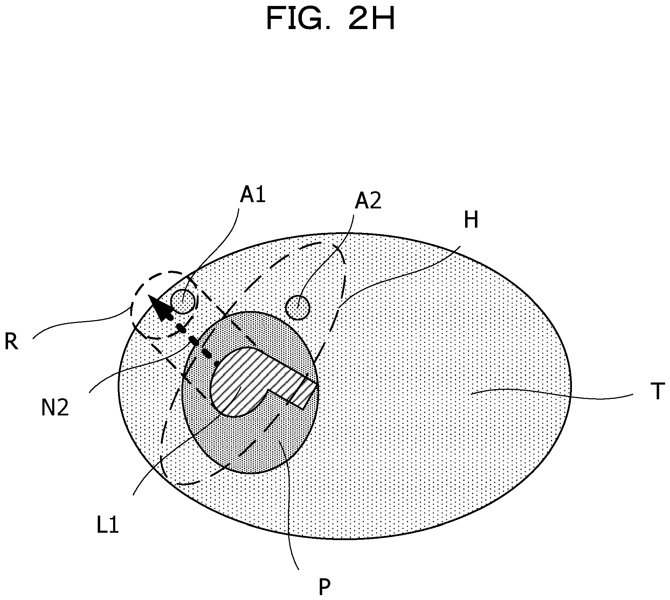

Next, in step S6, an atom (A2) of the target molecule existing on a surface H including the plane obtained from the ring structure of the obtained oriented binding calculation object molecule L1, the atom (A2) having a minor fluctuation when the binding site is obtained by the molecular dynamics, is selected (FIG. 2H).

### <<Step S7>>

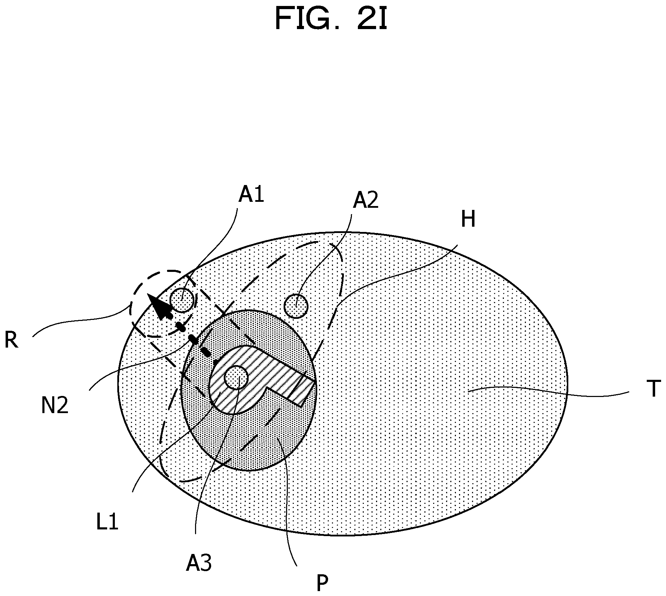

Next, in step S7, the center (A3) of the ring structure of the obtained oriented binding calculation object molecule L1 is obtained (FIG. 2I).

### <<Step S8>>

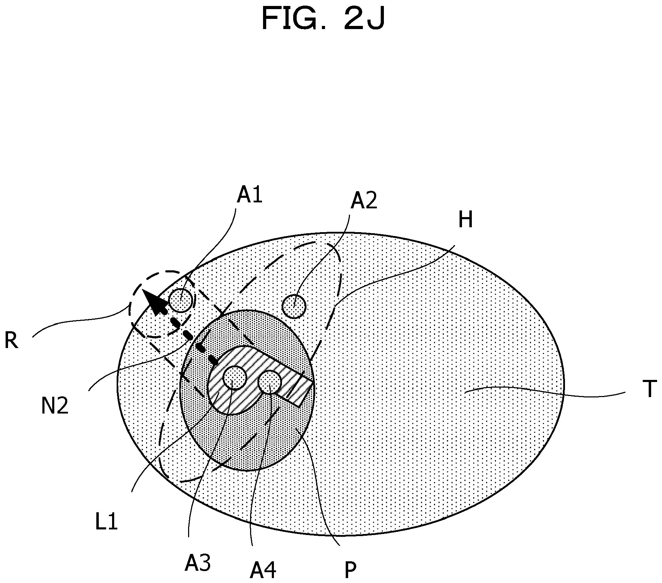

Next, in step S8, an atom (A4) that does not overlap with the center (A3) is selected from among the atoms included in the fragment of the obtained oriented binding calculation object molecule L1 (FIG. 2J).

### <<Step S9>>

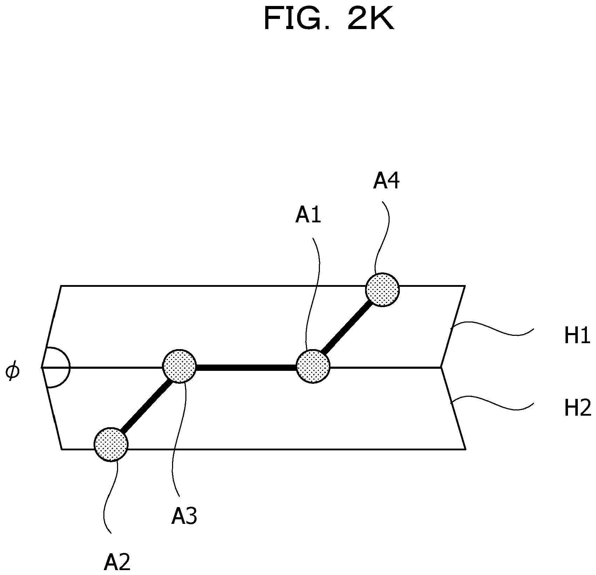

Next, in step S9, a dihedral angle ϕ formed by a plane H1 formed by the atom (A1), the center (A3), and the atom (A4) and a plane H2 formed by the atom (A1), the atom (A2), and the center (A3) is determined as a collective coordinate (FIG. 2K).

Note that an order of the steps S5, S6, and S7 is not particularly limited and can be appropriately selected according to a purpose.

A direction (D) of a straight line connecting the atom (A1) and the center (A3) is close to the normal direction (N2). In other words, by setting the obtained dihedral angle ϕ as a collective coordinate, it is possible to search for the stable binding structure (binding pose) around the direction (D) substantially orthogonal to the plane obtained from the ring structure of the binding calculation object molecule as a rotation axis.

In general, in the binding site of the target molecule, a surface of the ring structure of the binding calculation object molecule strongly interacts with the target molecule. In this way, the stable binding structure (binding pose) between the target molecule and the binding calculation object molecule can be obtained. Therefore, when the stable binding structure (binding pose) is obtained, the stable binding structure (binding pose) is more easily obtained by rotating the ring structure of the binding calculation object molecule in the plane direction.

Therefore, by using the method of determining the collective coordinate according to the disclosure, it is possible to obtain the collective coordinate suitable for searching for the stable binding structure (binding pose) without using the experimental information.

The method of determining the collective coordinate can be implemented by using a general computer system including, for example, a central processing unit (CPU), a random access memory (RAM), a hard disk, various peripheral devices, and the like (for example, various network servers, workstations, personal computers, and the like).

### (Method of Calculating Stable Binding Structure)

A method of calculating a stable binding structure related to the technology according to the disclosure is a method of calculating a stable binding structure between the target molecule and the binding calculation object molecule by using the collective coordinate determined by the method of determining the collective coordinate according to the disclosure.

The method of calculating the stable binding structure is performed, for example, by metadynamics. For example, a second stable binding structure that is more stable than a first stable binding structure is searched by metadynamics by using the collective coordinate from the first stable binding structure between the target molecule and the binding calculation object molecule.

The metadynamics is one of tabu search methods, and is a method of suppressing an existence probability in an once visited region to achieve a smooth probability distribution on coordinates by placing a potential proportional to the existence probability on coordinate axes (by imparting a penalty function).

In other words, the metadynamics is a method of smoothing a free energy surface by adding minute potentials one after another to a free energy curved surface (minimum) of a system by a penalty function.

By using the metadynamics, it is possible to increase a probability that an event occurs that rarely occurs in normal cases.

A specific method of the metadynamics when the metadynamics is used can be performed, for example, with reference to the document (Francesco Luigi Gervasio, Alessandro Laio, and Michele Parrinello, J. AM. CHEM. SOC. 2005, 127, 2600-2607).

The search for the second stable binding structure using the metadynamics can be performed, for example, by imparting a penalty function (penalty potential) to potential energy of a binding structure in a snapshot in molecular dynamics simulation of the target molecule and the binding calculation object molecule.

The penalty function is not particularly limited and can be appropriately selected according to a purpose. However, the penalty function is usually a Gaussian distribution type function.

The width and height of parameters of the penalty function are not particularly limited and can be appropriately selected according to a purpose.

The penalty function is usually imparted a plurality of times. A frequency (time interval) for imparting the penalty function is not particularly limited and can be appropriately selected according to a purpose. In other words, the penalty function may be imparted for each time step in the molecular dynamics simulation, or the penalty function may be imparted for each unit including several time steps. At this time, parameters of the penalty function used are preferably fixed.

The method for calculating a stable binding structure can be implemented by using a general computer system including, for example, a central processing unit (CPU), a random access memory (RAM), a hard disk, various peripheral devices, and the like (for example, various network servers, workstations, personal computers, and the like).

### (Program)

The program according to the disclosure is a program that causes a computer to execute the method of determining the collective coordinate according to the disclosure.

A preferred aspect in the execution of the method of determining the collective coordinate is the same as a preferred aspect in the method of determining the collective coordinate.

Another aspect of the program according to the disclosure is a program that causes a computer to execute the method of calculating the stable binding structure according to the disclosure.

A preferred aspect in the execution of the method of calculating a stable binding structure is the same as a preferred aspect in the method of calculating a stable binding structure.

The program can be created using various known programming languages according to the configuration of a computer system used, the type and version of an operating system, and the like.

The program may be recorded on a recording medium such as an internal hard disk or an external hard disk, or may be recorded on a recording medium such as a compact disc read only memory (CD-ROM), a digital versatile disk read only memory (DVD-ROM), a magneto-optical (MO) disk, or a universal serial bus (USB) memory [USB flash drive], for example. In a case where the program is recorded on a recording medium such as a CD-ROM, a DVD-ROM, an MO disk, or a USB memory, the program can be directly used through a recording medium reader included in the computer system, or be installed into a hard disk and be then used, as needed. Furthermore, the program may be recorded in an external storage region (another computer or the like) that is accessible from the computer system through an information communication network, and this program may be directly used from the external storage region through an information communication network, or be installed into a hard disk and then be used, as needed.

The program may be divided into respective processes, and be recorded on a plurality of recording media.

### (Computer-Readable Recording Medium)

A computer-readable recording medium according to the disclosure records the disclosed program.

The computer-readable recording medium is not limited to any particular medium, and may be appropriately selected according to a purpose. For example, examples of the computer-readable recording medium include an internal hard disk, an external hard disk, a CD-ROM, a DVD-ROM, an MO disk, a USB memory, or the like.

The recording medium may be a plurality of recording media on which the program that is divided for each optional process is recorded.

### (Collective Coordinate Determination Device)

A collective coordinate determination device according to the disclosure includes at least a binding site search unit, a normal direction (N1) search unit, a binding calculation object molecule search unit, an atom (A1) selection unit, an atom (A2) selection unit, a center (A3) search unit, an atom (A4) selection unit, and a collective coordinate determination unit and further includes other units as needed.

The collective coordinate determination device is a collective coordinate determination device that determines a collective coordinate capable of setting a search space of a stable binding structure (binding pose) when a target molecule is bound to a binding calculation object molecule.

The method of determining the collective coordinate can be performed, for example, by the collective coordinate determination device.

The binding site search unit obtains the binding site of the binding calculation object molecule in the target molecule by molecular dynamics by using a fragment which is a part or all of the binding calculation object molecule and includes a ring structure.

The normal direction (N1) search unit obtains a normal direction (N1) with respect to a plane obtained from the ring structure of the fragment existing at coordinates where the fragment frequently appears in the binding site when the binding site is obtained by the molecular dynamics.

The binding calculation object molecule search unit obtains the binding calculation object molecule which is in the binding site and does not overlap with the target molecule, the binding calculation object molecule being oriented so that a normal direction (N2) with respect to the plane obtained from the ring structure of the fragment of the binding calculation object molecule is overlapped with the normal direction (N1).

The atom (A1) selection unit selects an atom (A1) of the target molecule in a columnar region obtained by moving the ring structure of the obtained oriented binding calculation object molecule to the normal direction (N2), the atom (A1) having a minor fluctuation when the binding site is obtained by the molecular dynamics.

The atom (A2) selection unit selects an atom (A2) of the target molecule existing on a surface including the plane obtained from the ring structure of the obtained oriented binding calculation object molecule, the atom (A2) having a minor fluctuation when the binding site is obtained by the molecular dynamics.

The center (A3) search unit obtains a center (A3) of the ring structure of the obtained oriented binding calculation object molecule.

The atom (A4) selection unit selects an atom (A4) that does not overlap with the center (A3) from among the atoms included in the fragment of the obtained oriented binding calculation object molecule.

The collective coordinate determination unit determines a dihedral angle formed by a plane formed by the atom (A1), the center (A3), and the atom (A4) and a plane formed by the atom (A1), the atom (A2), and the center (A3) as a collective coordinate.

### (Stable Binding Structure Calculation Device)

A stable binding structure calculation device related to the technology according to the disclosure includes at least the collective coordinate determination device according to the disclosure and a stable binding calculation unit and further includes other units as needed.

A method of calculating the stable binding structure can be performed, for example, by the stable binding structure calculation device.

The stable binding calculation unit calculates a stable binding structure between the target molecule and the binding calculation object molecule by using the collective coordinate determined by the collective coordinate determination unit.

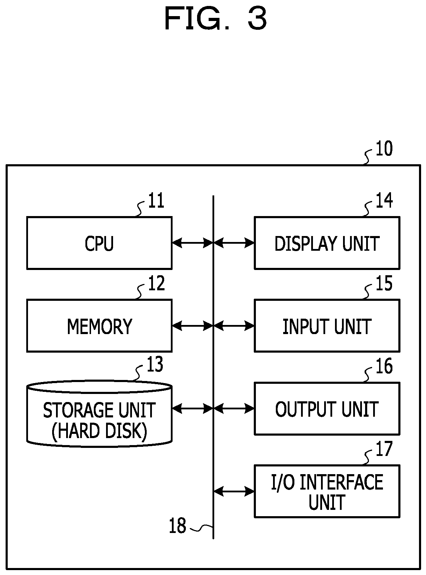

FIG. 3 illustrates an exemplary configuration of a collective coordinate determination device according to the disclosure.

A collective coordinate determination device 10 includes, for example, a CPU 11 (calculation unit), a memory 12, a storage unit 13, a display unit 14, an input unit 15, an output unit 16, an I/O interface unit 17, or the like that are connected to each other via a system bus 18.

The central processing unit (CPU) 11 performs arithmetic operations (such as four arithmetic operations and comparison operations), hardware and software operation control, and the like.

The memory 12 is a memory such as a random access memory (RAM) or a read only memory (ROM). The RAM stores an operating system (OS), an application program, and the like read from the ROM and the storage unit 13 and functions as a main memory and a work area of the CPU 11.

The storage unit 13 is a device that stores various types of programs and data, and may be a hard disk, for example. The storage unit 13 stores a program executed by the CPU 11, data necessary for executing the programs, an OS, and the like.

The program is stored in the storage unit 13, loaded into a RAM (main memory) of the memory 12, and executed by the CPU 11.

The display unit 14 is a display device, and may be a display device such as a CRT monitor or a liquid crystal panel, for example.

The input unit 15 is an input device for various types of data, and may be a keyboard, a pointing device (such as a mouse or the like), or the like, for example.

The output unit 16 is an output device for various types of data, and may be a printer, for example.

The I/O interface unit 17 is an interface for connecting various external devices. For example, the I/O interface unit 17 enables inputting/outputting of data into/from a CD-ROM, a DVD-ROM, an MO disk, a USB memory, or the like.

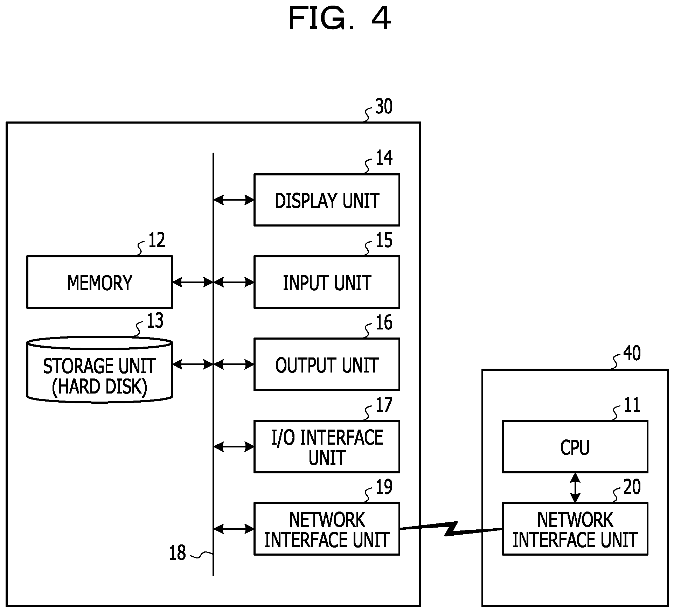

FIG. 4 illustrates another exemplary configuration of the collective coordinate determination device according to the disclosure.

The exemplary configuration in FIG. 4 is a cloud type exemplary configuration, in which the CPU 11 is independent of the storage unit 13 and the like. In this exemplary configuration, a computer 30 that includes the storage unit 13 and the like is connected to a computer 40 that includes the CPU 11 via network interface units 19 and 20.

The network interface units 19 and 20 are hardware that performs communication using the Internet.

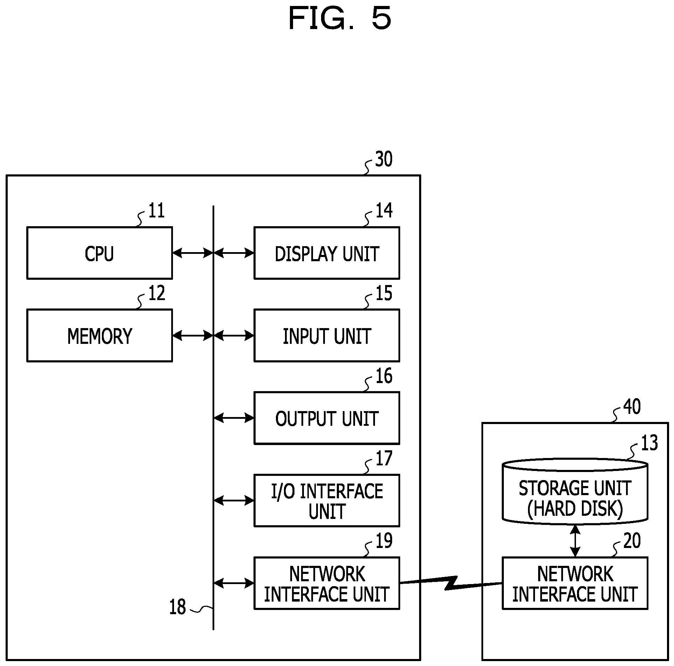

FIG. 5 illustrates still another exemplary configuration of the collective coordinate determination device according to the disclosure.

The exemplary configuration in FIG. 5 is a cloud type exemplary configuration, in which the storage unit 13 is independent of the CPU 11 and the like. In this exemplary configuration, the computer 30 that includes the CPU 11 and the like is connected to the computer 40 that includes the storage unit 13 via the network interface units 19 and 20.

### [Embodiments]

Hereinafter, the technology according to the disclosure will be described. However, the disclosed technology is not limited to the following embodiment.

### (First Embodiment)

For a human coagulation factor Xa (PDB crystal structure 1NFX) that is a protein to be calculated and a ligand (6-chlorobenzothiophene-2-ol), a collective coordinate used to search for a binding pose was determined. Specifically, the following method was performed along the flowchart in FIG. 1.

### <Step S1>

First, a fragment used for the method of determining the collective coordinate was determined from a ligand.

Because this ligand had a single ring structure and was a small molecule, the fragment was assumed to have the same structure as the ligand. In other words, an entire ligand was assumed as a fragment. Note that a ring structure of the ligand is a benzothiophene ring.

### <Step S2>

Next, a binding site of the ligand in the protein was obtained by the molecular dynamics (MD) by using the fragment.

The molecular dynamics was performed on the protein in a fragment solution (concentration 1 M) including the plurality of fragments, and a region of the protein where the fragments exist at a relatively high concentration during the molecular dynamics was assumed as a binding site. This binding site was located at the same position as a binding site of a 1NFX known inhibitor (RTR). Therefore, the next step was proceeded by using this binding site.

### <Step S3>

Next, the normal direction (N1) with respect to a plane obtained from the ring structure of the fragment at the coordinates of the fragment where the fragment frequently appears in the binding site when the binding site is obtained by the molecular dynamics was obtained.

### <Step S4>

Next, the ligand that is in the binding site and does not overlap with the protein, the ligand being oriented so that the normal direction (N2) with respect to the plane obtained from the ring structure of the fragment of the ligand is overlapped with the normal direction (N1), was obtained. However, in the present embodiment, because the ligand and the fragment have the same structure, a position where the fragment frequently appears was set as a ligand position.

### <Step S5>

Next, an atom (A1) of the protein in a columnar region obtained by moving the ring structure of the obtained oriented ligand to the normal direction (N2), the atom (A1) having a minor fluctuation when the binding site is obtained by the molecular dynamics, was selected. The selected atom (A1) is a nitrogen atom of ATOM 2831 N GLN A 19 in the PDB crystal structure 1NFX data.

### <Step S6>

Next, an atom (A2) of the protein existing on a surface including a plane obtained from the ring structure of the obtained oriented ligand, the atom (A2) having a minor fluctuation when the binding site is obtained by the molecular dynamics, was selected. The selected atom (A2) is a nitrogen atom of ATOM 3222 N CYS A 220 in the PDB crystal structure 1NFX data.

### <Step S7>

Next, a center (A3) of the ring structure of the obtained oriented ligand was obtained. The obtained center (A3) is center coordinates of LIG (x, y, z) = (28.1734, 27.7919, 35.4246) in the PDB crystal structure 1NFX data.

### <Step S8>

Next, an atom (A4) that does not overlap with the center (A3) was selected from among atoms included in the fragment of the obtained oriented ligand. The selected atom (A4) is a chlorine atom of ATOM 4426 cl LIG C 246 in the PDB crystal structure 1NFX data.

### <Step S9>

Next, a dihedral angle ϕ (A2 - A3 - A1 - A4) formed by a plane H1 formed by the atom (A1), the center (A3), and the atom (A4) and a plane H2 formed by the atom (A1), the atom (A2), and the center (A3) was determined as a collective coordinate.

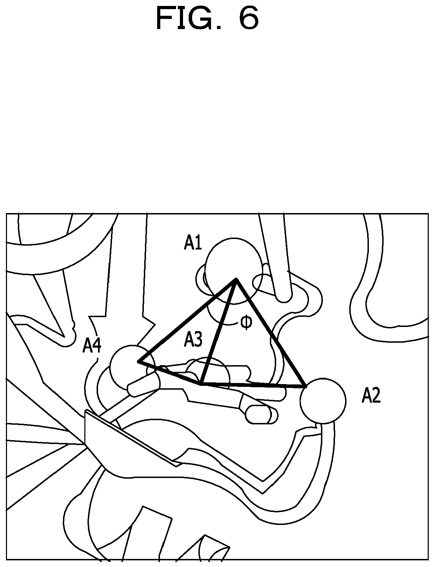

When the atom (A1), the atom (A2), the center (A3), and the atom (A4) are illustrated in the figure, positions of spheres illustrated in FIG. 6 are obtained.

Then, when the binding pose was searched by metadynamics by using the obtained collective coordinate, a normal operation was confirmed. In other words, it was confirmed that the method of determining the collective coordinate according to the disclosure was effective in the search for the binding pose. Note that the metadynamics was performed by using gromacs patched with plumed.

This result indicates that it is possible to determine the collective coordinate used to express a search space necessary for efficiently searching for the binding pose with no experimental information of the binding site by using the method of determining the collective coordinate according to the disclosure.

Note that there is a case where it takes a year only to, for example, crystallize a complex of a protein and a ligand to obtain the experimental information. However, in the method of determining the collective coordinate according to the above described disclosure, most of required time is used to search for a binding site, and this can be achieved by performing molecular dynamics for several days.

### REFERENCE SIGNS LIST

- 10: collective coordinate determination device

- 11: CPU
- 12: memory
- 13: storage unit
- 14: display unit
- 15: input unit
- 16: output unit
- 17: I/O interface unit
- 18: system bus
- 19: network interface unit
- 20: network interface unit
- 30: computer
- 40: computer

## Claims

1. A method of determining a collective coordinate, by using a computer, that determines a collective coordinate capable of setting a search space of a stable binding structure when a target molecule is bound to a binding calculation object molecule, the method comprising:
a process of obtaining a binding site of the binding calculation object molecule in the target molecule by molecular dynamics by using a fragment, including a ring structure, that is a part or all of the binding calculation object molecule;
a process of obtaining a normal direction (N1) with respect to a plane obtained from the ring structure of the fragment at coordinates of the fragment where the fragment frequently appears in the binding site when the binding site is obtained by the molecular dynamics;
a process of obtaining the binding calculation object molecule that is in the binding site and does not overlap with the target molecule, the binding calculation object molecule being oriented so that a normal direction (N2) with respect to a plane obtained from the ring structure of the fragment of the binding calculation object molecule is overlapped with the normal direction (N1);
a process of selecting an atom (A1) of the target molecule in a columnar region obtained by moving the ring structure of the obtained oriented binding calculation object molecule to the normal direction (N2), the atom (A1) having a minor fluctuation when the binding site is obtained by the molecular dynamics;
a process of selecting an atom (A2) of the target molecule existing on a surface including a plane obtained from the ring structure of the obtained oriented binding calculation object molecule, the atom (A2) having a minor fluctuation when the binding site is obtained by the molecular dynamics;
a process of obtaining a center (A3) of the ring structure of the obtained oriented binding calculation object molecule;
a process of selecting an atom (A4) that does not overlap with the center (A3) from among atoms included in the fragment of the obtained oriented binding calculation object molecule; and
a process of determining a dihedral angle formed by a plane formed by the atom (A1), the center (A3), and the atom (A4) and a plane formed by the atom (A1), the atom (A2), and the center (A3) as a collective coordinate.

2. The method of determining a collective coordinate according to claim 1, wherein
the process of obtaining the binding site is performed by performing the molecular dynamics on the target molecule in a fragment solution including a plurality of the fragments and assuming a region of the target molecule where the fragments exist at a relatively high concentration during the molecular dynamics as a binding site.

3. The method of determining a collective coordinate according to claim 1 or 2, wherein
the coordinates in the process of obtaining the normal direction (N1) are coordinates where a number of the fragments exist in the binding site when a plurality of snapshots obtained by the molecular dynamics is superimposed.

4. The method of determining a collective coordinate according to any one of claims 1 to 3, wherein
a fluctuation of the atom (A1) selected in the process of selecting the atom (A1) is a fluctuation minor than vibration caused by heat at 25°C.

5. The method of determining a collective coordinate according to any one of claims 1 to 4, wherein
a fluctuation of the atom (A2) selected in the process of selecting the atom (A2) is a fluctuation minor than vibration caused by heat at 25°C.

6. The method of determining a collective coordinate according to any one of claims 1 to 5, wherein
the binding calculation object molecule is a drug candidate molecule.

7. A program for causing a computer to determine a collective coordinate capable of setting a search space of a stable binding structure when a target molecule is bound to a binding calculation object molecule, the program causing a computer to execute:
a process of obtaining a binding site of the binding calculation object molecule in the target molecule by molecular dynamics by using a fragment, including a ring structure, that is a part or all of the binding calculation object molecule;
a process of obtaining a normal direction (N1) with respect to a plane obtained from the ring structure of the fragment at coordinates of the fragment where the fragment frequently appears in the binding site when the binding site is obtained by the molecular dynamics;
a process of obtaining the binding calculation object molecule that is in the binding site and does not overlap with the target molecule, the binding calculation object molecule being oriented so that a normal direction (N2) with respect to a plane obtained from the ring structure of the fragment of the binding calculation object molecule is overlapped with the normal direction (N1);
a process of selecting an atom (A1) of the target molecule in a columnar region obtained by moving the ring structure of the obtained oriented binding calculation object molecule to the normal direction (N2), the atom (A1) having a minor fluctuation when the binding site is obtained by the molecular dynamics;
a process of selecting an atom (A2) of the target molecule existing on a surface including a plane obtained from the ring structure of the obtained oriented binding calculation object molecule, the atom (A2) having a minor fluctuation when the binding site is obtained by the molecular dynamics;
a process of obtaining a center (A3) of the ring structure of the obtained oriented binding calculation object molecule;
a process of selecting an atom (A4) that does not overlap with the center (A3) from among atoms included in the fragment of the obtained oriented binding calculation object molecule; and
a process of determining a dihedral angle formed by a plane formed by the atom (A1), the center (A3), and the atom (A4) and a plane formed by the atom (A1), the atom (A2), and the center (A3) as a collective coordinate.

8. The program according to claim 7, wherein
the process of obtaining the binding site is performed by performing the molecular dynamics on the target molecule in a fragment solution including a plurality of the fragments and assuming a region of the target molecule where the fragments exist at a relatively high concentration during the molecular dynamics as a binding site.

9. The program according to claim 7 or 8, wherein
the coordinates in the process of obtaining the normal direction (N1) are coordinates where a number of the fragments exist in the binding site when a plurality of snapshots obtained by the molecular dynamics is superimposed.

10. The program according to any one of claims 7 to 9, wherein
a fluctuation of the atom (A1) selected in the process of selecting the atom (A1) is a fluctuation minor than vibration caused by heat at 25°C.

11. The program according to any one of claims 7 to 10, wherein
a fluctuation of the atom (A2) selected in the process of selecting the atom (A2) is a fluctuation minor than vibration caused by heat at 25°C.

12. The program according to any one of claims 7 to 11, wherein
the binding calculation object molecule is a drug candidate molecule.

13. A collective coordinate determination device that determines a collective coordinate capable of setting a search space of a stable binding structure when a target molecule is bound to a binding calculation object molecule, the collective coordinate determination device comprising:
a binding site search unit configured to obtain a binding site of the binding calculation object molecule in the target molecule by molecular dynamics by using a fragment, including a ring structure, that is a part or all of the binding calculation object molecule;
a normal direction (N1) search unit configured to obtain a normal direction (N1) with respect to a plane obtained from the ring structure of the fragment at coordinates of the fragment where the fragment frequently appears in the binding site when the binding site is obtained by the molecular dynamics;
a binding calculation object molecule search unit configured to obtain the binding calculation object molecule that is in the binding site and does not overlap with the target molecule, the binding calculation object molecule being oriented so that a normal direction (N2) with respect to a plane obtained from the ring structure of the fragment of the binding calculation object molecule is overlapped with the normal direction (N1);
an atom (A1) selection unit configured to select an atom (A1) of the target molecule in a columnar region obtained by moving the ring structure of the obtained oriented binding calculation object molecule to the normal direction (N2), the atom (A1) having a minor fluctuation when the binding site is obtained by the molecular dynamics;
an atom (A2) selection unit configured to select an atom (A2) of the target molecule existing on a surface including a plane obtained from the ring structure of the obtained oriented binding calculation object molecule, the atom (A2) having a minor fluctuation when the binding site is obtained by the molecular dynamics;
a center (A3) search unit configured to obtain a center (A3) of the ring structure of the obtained oriented binding calculation object molecule;
an atom (A4) selection unit configured to select an atom (A4) that does not overlap with the center (A3) from among atoms included in the fragment of the obtained oriented binding calculation object molecule; and
a collective coordinate determination unit configured to determine a dihedral angle formed by a plane formed by the atom (A1), the center (A3), and the atom (A4) and a plane formed by the atom (A1), the atom (A2), and the center (A3) as a collective coordinate.

14. The collective coordinate determination device according to claim 13, wherein
the binding site is obtained by the binding site search unit by performing the molecular dynamics on the target molecule in a fragment solution including a plurality of the fragments and assuming a region of the target molecule where the fragments exist at a relatively high concentration during the molecular dynamics as a binding site.

15. The collective coordinate determination device according to claim 13 or 14, wherein
the coordinates when the normal direction (N1) is obtained by the normal direction (N1) search unit are coordinates where a number of the fragments exist in the binding site when a plurality of snapshots obtained by the molecular dynamics is superimposed.

16. The collective coordinate determination device according to any one of claims 13 to 15, wherein
a fluctuation of the atom (A1) selected by the atom (A1) selection unit is a fluctuation minor than vibration caused by heat at 25°C.

17. The collective coordinate determination device according to any one of claims 13 to 16, wherein
a fluctuation of the atom (A2) selected by the atom (A2) selection unit is a fluctuation minor than vibration caused by heat at 25°C.

18. The collective coordinate determination device according to any one of claims 13 to 17, wherein
the binding calculation object molecule is a drug candidate molecule.
